# EUROPEAN PATENT SPECIFICATION

(11) **EP 2 309 917 B1**
(45) Date of publication and mention of the grant of the patent: **05.09.2018**
(21) Application number: 09774696.0
(22) Date of filing: 03.07.2009
(51) Int. Cl.: A61B 5/00, G06F 19/00

(54) **DEVICE THAT FOR INTERACTIVELY MANAGING A TREATMENT FOR GLYCEMIC LEVEL CONTROL IN A DIABETIC PATIENT**
VORRICHTUNG FÜR INTERAKTIVE KONTROLLE EINER BEHANDLUNG ZUR BLUTZUCKERKONTROLLE BEI EINEM DIABETIKER
DISPOSITIF POUR GÉRER DE FAÇON INTERACTIVE UN TRAITEMENT DE RÉGULATION DE LA GLYCÉMIE CHEZ UN PATIENT DIABÉTIQUE

(30) Priority: 10.07.2008 IT BO20080430
(43) Date of publication of application: 20.04.2011
(73) Proprietor: BG Informatica S.R.L., 63039 San Bernedetto del Tronto (AP) (IT)
(72) Inventor: VESPASIANI, Giacomo, 63039 San Benedetto del Tronto (AP) (IT)
(74) Representative: Ruzzu, Giammario
(86) International application number: PCT/IB2009/006174
(87) International publication number: WO 2010/004406

(56) References cited:
- EP-A- 0 970 655
- EP-A- 1 677 226
- WO-A-03/015838
- WO-A-03/036409
- WO-A-03/041425
- US-A- 4 731 726
- US-A1- 2003 125 612
- US-A1- 2007 294 360
- US-A1- 2008 300 534
- US-A1- 2009 138 207
- US-B1- 6 656 114

## Description

### BACKGROUND OF THE INVENTION

The present invention relates to the technical field concerning telemedicine applied to monitoring and therapeutic intervention in the diabetic patients.

In particular, the invention relates to a method, and a device that carries out the method, for interactive definition and management of a therapy for the blood glucose monitoring in such patients, and for keeping it within the limits set by the sanitary protocols for such pathologies.

### BRIEF DESCRIPTION OF THE PRIOR ART

It is known that diabetes patients must measure the blood glucose level periodically, even daily or more times a day, in various physical and metabolic activity situations. This is crucial for the doctor in charge to establish, and to modify, if necessary, a specific therapeutic program for each patient. Such a program calculates the administration of insulin doses irrespective of the definition of the quantity of carbohydrates that the patient can consume during the day.

Although it depends on the quantity of carbohydrates and on different factors, for example, physical activity performed by the patient during the day, the insulin dosage can be calculated on the basis of the glycemia of the previous days, according to rules, which the doctor can adjust over time for each patient.

Generally, a close collaboration between the patient and his doctor is necessary. The first one must learn to follow different behavior rules prescribed by the latter, which include exactly the blood glucose self-monitoring frequency, calculation of a daily diet that allows to assimilate the right quantity of carbohydrates and performing of a constant and planned physical activity. Furthermore, he must inform periodically his doctor about the trend of the glycemic values measured by the self-monitoring and the glycemia values resulting from the insulin dose actually taken. On the basis of such data, the doctor confirms or re-formulates the insulin dose, or otherwise, provides the patient with instruments for calculating such a dose.

In particular, in case of diabetes patients of type 2 (insulin-deficient), maintaining the correct blood glucose control is as important as in case of diabetes patients of type 1 (insulin-lacking).

There are different therapeutic options for the type 2 diabetic patient: Metformin, sulphanylureas, glinides, (oral hypoglycaemic drugs) and insulin; the latter, although the most efficacious, is used late in the natural history of the diabetes treatment. Such delay in the change of the drug type up to the insulin is called therapeutic inertia and can last from 5 to 8 years. Due to this therapeutic inertia, in the past years associated with glycated hemoglobin levels higher than the target (7 or 6.5% according to different standards) in the beginning of the insulin therapy, many patients already suffer from the diabetes complications (eyes, kidney, heart....).

When the therapeutic inertia period, even too long, is passed and the insulin therapy is begun, the prescribed insulin dose is very often insufficient, and particularly, it remains fixed over time and does not follow the patient real need, that, as already said, follows an extremely personal trend.

As a result, at present, a mean value of the glycated hemoglobin among the type 2 diabetic patients treated with insulin in Italy is over 8.3%, which puts them at considerable risk of complications, particularly cardiovascular complications. This occurs in spite of the fact that the self-monitoring of glycemia at home is common also with the type 2 diabetic patients.

There are different reasons for which the insulin dose cannot be optimized to reach the target of 7% of the glycated hemoglobin.

First of all, the type 2 patient is often elderly, within the medium age bracket of more than 60, and, although he is able to perform autonomously the glycemic control in finger tip (glycemic self-monitoring), on the average he finds it very difficult to learn what to do with the glycemia he had measured.

Moreover, the diabetes specialist visits this type of patient every 4-6 months and he must do his new prescription for a subsequent equal period on the basis of the data collected by the patient during the last 3-7 days. Therefore, he remains powerless faced with the variations that occur in such a long period of time, i.e. with respect to hypoglycemia or hyperglycemia situations that inevitably take place.

On the other hand, the general practitioner, who visits regularly over 60% of the above mentioned patients, is not experienced in the methods of changing the insulin dose in relation to different situations that happen to the same patients. Actually, there are different algorithms for adjustment of the insulin dose, however they must be applied regularly, day by day, on the basis of the diabetic patient's lifestyle and on his significant parameters. In addition, it is suggested that such algorithms be applied selectively, depending on the single patient response, or even periodically modified or substituted according to changing of the patient's condition. Most patients cannot cope with its correct management.

It is necessary to take into consideration also the fact that the number of the type 2 diabetic patients is very high, and moreover, in clear increase (about 4-6% of the population), therefore, from a statistic point of view, the probability that the patients are not able to understand and apply the relatively complicated "mechanisms" of the glycemic monitoring, or in any case, that they do not apply them with due regularity, is very high.

On the other hand, in view of the patients number and typology, also the presence of methodologies that include the transmission to the diabetes specialist of information related to the blood glucose of each patient each time he/she performs self-monitoring, has appeared substantially non practicable, or in any case, has not influenced considerably the global reduction of the therapeutic inertia times and of the subsequent mean value of the glycated hemoglobin level in the type 2 diabetic patients.

In order to improve the patient's capacity in self-monitoring their blood glucose level, there have been proposed several portable devices for acquiring the glycemic level from a patient, for storing its value in a memory area, and for sending the acquired data to some remote database, so as it is available to, e.g., a medical professional.

WO 03/015838 to Novo Nordisk A/S discloses a portable medical device provided with a medical transducer, a body fluid analyzer and a blood glucose monitor for reading and storing the glycemic level, processing means for preparing a set of the patient's data, and a communication device for sending said data to a central server, in order to make them accessible to a medical professional or to a patient's relative.

Moreover, both EP 1 677 226 to Vespasiani, Giacomo and WO 03/036409 to France Telecom teach how a medical professional can retrieve data coming from a portable device used by a diabetic patient and send messages to the same patient, by means of a SMS, MMS or E-Mail, in order to provide him with updated information about a diet composition or about a new insulin dosage.

However, the known art methods and devices do not address the problem of significantly reducing the therapeutic inertia period, nor how to adjust the insulin dosage without any intervention of the same patient.

### OBJECTS OF THE INVENTION

An object of the present invention is to propose, for both the patient and diabetes specialist, an instrument for acquiring information for the definition of the insulin dose, which allows the patient to know each time the substantially optimal insulin dose for his actual situation, and which allows the doctor to monitor constantly the patient's trend on the short and long run, without increasing unmanageably his workload.

Another object of the present invention is to provide the diabetic patient with an instrument which gives him, day by day, the correct insulin dose, in terms of his real needs, however without having him do calculations or other operations different from those done usually for the self-monitoring of blood glucose.

A further object of the present invention is to provide the diabetic patient with an instrument capable of suggesting him most suitable moments for such self-monitoring, on the basis of his actual situation and needs of the computing algorithm of the insulin dose that he is following in that moment.

A still further object of the present invention is to provide the diabetes specialist with an instrument for constant situation control of each patient, which instrument manages to point out the beginning of anomalous trends of the patient's blood glucose, short or long, communicating them to the diabetes specialist without any patient's voluntary intervention.

### SUMMARY OF THE INVENTION

The above mentioned objects are all obtained, in accordance with the contents of the claims.

### DESCRIPTION OF PREFERRED EMBODIMENTS OF THE INVENTION

The characteristics of the invention, as they will appear from the claims, are pointed out in the following detailed description.

According to the present invention, a diabetic patient, for example a type 2 diabetic patient, is suitably equipped with an integrated device for the interactive definition and management of a treatment for the blood glucose self-monitoring, aimed at assisting him with following a personalized treatment method, under constant control of his diabetes specialist.

The example of the type 2 diabetic patient (insulin-deficient) is particularly significant, although not limiting, as the following considerations can be equally made for the type 1 patient (insulin-lacking). Actually, such patient, as already mentioned previously, is in the average rather elderly, and in any case, he is statistically less prone to assimilate and perform, with the necessary constancy and accuracy, all the operations necessary to maintain the control and the memory (with the prompt registration of the performed actions and of the periodically acquired data), no matter how able he is to understand quite easily the glucometer use for monitoring his blood glucose. Moreover, he tends to delay the communication of the data he has managed to register to his diabetes specialist and does not react with the dose change as the glycemia values vary.

Thus, this type of patient takes particular advantage of the use of the present integrated device. The aforesaid device includes essentially a module composed of a digital glucometer of conventional type, aimed at allowing the patient to measure his blood glucose value and at acquiring such value in digital code.

The device includes also a data processing unit, connected to the aforesaid digital glucometer and aimed at running a memorized program for managing device functions, and including rewritable permanent memory units, aimed at receiving and maintaining for a long time the instructions of the aforesaid management program and the data acquired during the device operation, as well as other data, whose origin and function will be better described later on.

The data processing unit is provided, according to the substantially known constructive schemes and techniques, also with a display device, composed of a liquid crystal display and a command and data input device, preferably limited to a number of keys that is not more than 2-3.

According to the invention, the integrated device includes also a simplified data transmitter and receiver module operating via a mobile telephone network, aimed at transmitting and receiving data on such telephone network, connected by means of standardized communication interfaces with the aforesaid data processing unit.

Fundamentally, the aforesaid transmitter/receiver module is composed of a kind of cellular telephone, without its vocal communication ability, but able to transmit and receive SMS, MMS, GPRS or other types of messages containing data or programs for computer. Modules of this type are commonly available, as a semi-finished industrial product, and are equipped with interfaces and connection protocols standardized to the microprocessor units of the type used in the digital glucometer, likewise available as semi-finished industrial products to be assembled and integrated in the glucometer design. Their internal structure, as well as the interfaces and the design knowledge necessary for their integration, belong one by one to the technical knowledge of an average skill electronic designer, and are not strictly relevant to the invention and thus they will not be described in a more detailed way later.

In the present integrated device, the transmitter/receiver module is aimed particularly at transmitting and receiving messages to/from a corresponding cellular device, either independent from or integrated in a computer, in any case connected to the aforesaid computer of a diabetes specialist who supervises the diabetic patient treatment. The aforesaid computer is supposed to be equipped with a message management program, that is program that analyzes the data sent by the patient, displays them and re-transmits updating information to the patient's self-monitoring integrated device, as it will be better explained later. At least one management program in known and available on the market, which carries out the above mentioned functions, it having been widely tested for a long time.

The above described modules of the integrated device are preferably assembled and contained in the same digital glucometer container, so the patient sees them as one of the typical glucometers he uses normally for his periodic blood glucose monitoring. However, its functionalities are much more complicated and comply with the operational modes for the management program described in the following.

Besides the normal user interface procedures, the treatment management program run on the self-monitoring device provides procedures interacting with the acquisition module of the suitably codified blood glucose value, aimed at storing such value in a corresponding permanent memory area.

The management program includes moreover a procedure aimed at implementing a periodic and personalized computing algorithm of each insulin dose to be administered to the patient. This calculation is made on the basis of information related to the last acquired blood glucose value, and a group of previous values related to the glycemia and the insulin doses actually taken previously by the patient. The algorithm defines also the time that must pass before acquiring a new blood glucose value.

In this connection there are different algorithms for insulin dosage, with both direct action and delayed action, known from the literature and widely used by the specialists. Other algorithms are also prepared by the same specialists, on the basis of their skill and convictions. Such algorithms, suitably personalized, are typically used to calculate the insulin dosage of each single patient, and possibly modified or substituted in relation to the results obtained in the medium-long period, that is from some weeks to some months.

Besides the above mentioned implementation procedure of the computing algorithm, the treatment management program includes a local management procedure, aimed at implementing a first short working cycle. It includes a sequence of instructions for interacting with the patient, aimed at informing the latter about the necessity to measure the blood glucose level, for directing the acquisition module of the blood glucose level and for storing of the acquired value in the memory area.

The above mentioned local procedure includes afterwards a sequence of instructions for running the aforesaid implementing procedure of the computing algorithm and for displaying the related insulin dose calculated for the patient. There are also instructions for monitoring the time passed from the last measurement and for the consequent re-running of the first local working cycle on the date and at the time calculated by the above mentioned computing algorithm.

Furthermore, the system sends automatically to the doctor the glycemic information, if they are beyond the mean value parameters, the time passed from the last contact and number of hypoglycemia measures, which the doctor himself can set for each patient and modify over time.

The local procedure can also contain instructions for the request, acquisition and storing of information related to the patient's diet, if the use of such information is provided by the computing algorithm being used.

For instance, the patient can be asked if he has or has not assimilated carbohydrates, immediately before the blood glucose level control, the result of which could influence the blood glucose value to be acquired. The management program of the self-monitoring treatment includes also a remote management procedure, aimed at implementing a second, long working cycle.

Such second, remote working cycle is aimed at being run by the management program at predetermined time limits, or coinciding with the occurrence of anomalous situations of the treatment trend.

In particular, the remote procedure includes sequences of instructions for reading, from the data area, of the patient's latest registered value of blood glucose level, of a group of values registered previously, for controlling a time limit, supplied by the control algorithm currently operational, and for running a further procedure on the aforesaid date. The latter includes extraction, from the memory areas, in which they have been stored by the computing algorithm, of a group of significant data of the diabetic patient treatment trend, related to the progressively assumed value of the patient's blood glucose level and to the corresponding insulin doses, provided by the computing algorithm. Other data contained in the group can be those related to the patient's nutritional habits, as well as those registered by means of the procedure that activates the already mentioned local working cycle.

Instruction sequences are also provided for subsequent forwarding of significant data to the transmitter/receiver module and for controlling it up to transmission of the aforesaid group of data to the remote transmission/reception device connected to the specialist's computer.

Activation procedure of the remote cycle includes also instructions for monitoring the foregoing transmitter/receiver module, for recognizing and acquiring messages possibly coming from the same module. Such messages contain generally a set of modified parameters for activation of the computing algorithm or of a new computing algorithm, suitably provided by the specialist on the basis of a check of the patient's blood glucose value temporal trend. They are suitably codified and can be recognized by a section of the above mentioned procedure aimed at installing a new set of parameters or the new algorithm in the permanent memory of the processing unit, so that afterwards they are implemented by the above mentioned activation procedure of the first local cycle.

The method for interactive definition and management of the treatment for the blood glucose level monitoring according to the invention consists substantially of the asynchronous and simultaneous execution, by the control unit of the integrated device, of the first local working cycle, that typically lasts a few hours, and of the second remote working cycle, that typically lasts from a few days to a few weeks, as described previously. In particular, the first local cycle includes the steps of asking the patient to do the blood glucose test by the glucometer and of acquiring from the latter a blood glucose value in digital code. Subsequently, the procedure is activated to implement the currently installed insulin dose computing algorithm, after having possibly asked the patient whether he had or had not recently taken carbohydrates. The aforesaid insulin dose, thus calculated on the basis of the last blood glucose value, of its trend in a previous predetermined period and of possible other acquired parameters, is shown on the display of the integrated device.

The frequency of the blood glucose level measurements, and consequently, the moment in which the patient is asked to do it, usually depends on the structure of the computing algorithm implemented in the integrated device, and therefore, the patient does not have to do tests at each meal, but only when the device asks him to do so.

Finally, the algorithm defines an activation date and time of an active phase of the second, remote working cycle, based on the peculiar needs depending on the patient general situation, defined by the specialist, and defines also a date and a time for the subsequent glycemic check. The first local cycle resumes its running on such date and at such time with the subsequent request to perform the check.

The second, remote work cycle includes the steps of reading of the last acquired blood glucose value, monitoring of the date and time defined for its active phase and performing of a series of work steps on such date and at such time.

The work steps include extraction of the above described group of data significant for the patient, codifying of the data in a conventional format, forwarding of the aforesaid significant data to the transmitter/receiver module and transmission of the data to the specialist's remote computer.

The transmitted data are subsequently acquired from the already mentioned display program resident in the doctor's computer. Afterwards, the doctor evaluates the transmitted data and decides whether to maintain or not the treatment parameters, whether to modify such parameters, or substitute the computing algorithm with a new or modified one.

The remote working cycle continues monitoring of the transmitter/receiver module, to verify possible receiving of a message containing suitably codified data.

This message is periodically transmitted by the specialist, if he decides to modify the treatment parameters or the computing algorithm.

If a message is received, a subsequent phase of the remote working cycle is started, which introduces the modified parameters or the algorithm to the memory area provided for them in the processing unit of the integrated device.

A further step of the aforesaid remote working cycle includes also evaluation of the patient's blood glucose level trend in a predetermined period of time and possible immediate activation of the data preparation and transmission step, if the trend is considered unstable, according to the parameters provided by the above mentioned computing algorithm.

Likewise, a further step, performed after each reading of the blood glucose value, includes preparation and immediate transmission of the patient's significant data, if the computing algorithm detects the presence of a serious hypoglycemia or hyperglycemia.

There are many advantages that the present invention allows to obtain with respect to the known managing methods and systems of the prior art. First of all, the management method organized according to two working cycles, substantially asynchronous and independent, on the one hand allows the patient to do his self-monitoring tests accurately and exactly, when it is decided by the particular computing algorithm, which has been prescribed to him in that moment by the specialist, and on the other hand, not to worry about all implications following each blood glucose measurement, related both to data registration and to supplying of further secondary information. Secondly, the patient is freed from the long-term tasks, such as periodic transmission of the registrations to his specialist, possible acquisition of treatment new modes and new doses, as well as application, and most of all, assimilation of such new modes and doses.

The device built according to the invention is advantageously capable of freeing the patient from all these operations of managing the data acquired by the glucometer, allowing him to think only about acquiring the blood glucose value by means of the same glucometer, which usually does not create him any difficulties.

The patient is also advantageously freed from all operations of manual data registration and transmission to the specialist, which in many cases was performed not correctly and with not optimal timing for the evaluation of the treatment efficacy.

It is understood that what above has been described as a pure, not limiting example. Therefore, possible changes and variants of the invention are to be considered within the protective scope of the present technical solution, as claimed below.

## Claims

1. A device for interactive definition and management of a treatment for controlling blood glucose level in a diabetic patient, said device including:
a digital glucometer, forming self-monitoring means, aimed at measuring the glycemic value of said patient and at storing it as a digital code, said digital glucometer comprising a data processing unit, which is adapted to run at least a treatment management program; a display, controlled by said data processing unit and electrically connected thereto;
a plurality of keys for input of data or commands, likewise electrically connected to said data processing unit;
said digital glucometer being provided with transmitter and receiver means, which are operating via a mobile telephone network and controlled by said data processing unit, for receiving and transmitting data from and to at least one corresponding remote reception/transmission device, connected to a computer accessible by a specialist supervising the aforesaid medical treatment of the diabetic patient; said treatment management program includes at least one local management procedure for implementing a first, shorter, local cycle, said local management procedure comprising instruction sequences for acquiring at least one blood glucose value of said patient by said self-monitoring means and for storing the value in a data area of said data processing unit, for calculation of an insulin dosage value to administer to said patient, by means of a computing algorithm, provided by the specialist, on the basis of acquired blood glucose value and of temporal trend of said blood glucose level in a previous period of time, for displaying said insulin dosage on said display, for defining of a date and time for the subsequent measurement of the blood glucose and instructions for warning said patient to perform said measurement in said date and on said time; said treatment management program further includes at least one remote management procedure for implementing a second, longer, remote cycle, said remote management procedure comprising sequences of instructions for reading, from said data area, last patient's blood glucose value, for periodical running of a procedure extracting a group of data significant for the patient, related to at least the patient's trend of the blood glucose values and to his insulin doses recommended on the basis of a computation made by said computing algorithm in a previous predetermined period, for transmitting of said group of significant data to a remote computer, accessible by said specialist, by means of said transmitter and receiver means, for possible receiving of a set of parameters modified on the basis of a deviation from said blood glucose trend with respect to the optimal one, for performing said computing algorithm, or a new computing algorithm, and for introducing said set of modified parameters or said new algorithm in said treatment management program, for applying the same in the above mentioned first local cycle; wherein said local management procedure further includes instructions for monitoring the time passed from the last measurement and for a consequent re-running of said first local working cycle on the date and at the time calculated by said computing algorithm, and wherein the first, shorter, local working cycle and the second, longer, remote working cycle are performed asynchronously and simultaneously.

2. A device according to claim 1, **characterized in that** said transmitter and receiver means is integrated in the structure of said digital glucometer.

3. A device according to claim 1, **characterized in that**, in said data processing unit, said treatment management program includes at least one procedure aimed at implementing the computing algorithm of the patient's insulin dosage, based on periodical information related to patient's blood glucose level and to insulin doses actually taken by said patient in a given previous period of time.

## Patentansprüche

1. Ein Gerät zur interaktiven Definition und Verwaltung einer Behandlung zur Kontrolle des Blutzuckerspiegels bei einem Diabetes-Patienten, wobei das Gerät die folgendes aufweist:
ein digitales Glukometer, das Selbstüberwachungsmittel bildet, die den glykämischen Wert des Patients messet und als digitalen Code speichert, wobei das digitale Blutzuckermessgerät eine Verarbeitungseinheit aufweist, die mindestens ein Behandlungsmanagementprogramm ausführt;
ein Display, das von der Datenverarbeitungseinheit gesteuert wird und elektrisch mit dem Gerät verbunden ist;
eine Vielzahl von Tasten zur Eingabe von Daten oder Befehlen, die ebenfalls elektrisch mit der Datenverarbeitungseinheit verbunden sind;
wobei das digitale Blutzuckermessgerät ist mit Sender- und Empfängermitteln ausgestattet ist, die über ein Mobilfunknetz arbeiten und von der Datenverarbeitungseinheit zum Empfangen und Senden von Daten von und zu mindestens einem entsprechenden Fernempfangs-/Sendegerät gesteuert werden, das an einen für einen Facharzt, der die oben genannte medizinische Behandlung des Patients überwacht, zugänglich Computer angeschlossen ist;
wobei das Behandlungsmanagementprogramm mindestens ein lokales Managementverfahren umfasst, das einen ersten, kürzeren lokalen Zyklus implementiert und Anweisungssequenzen zur Erfassung mindestens eines Blutzuckerwertes des Patients durch die Selbstüberwachungsmittel und zum Speichern des Wertes in einem Datenbereich der Datenverarbeitungseinheit umfasst, sowie für die Berechnung eines an den Patient zu verabreichen Insulindosierungswertes mittels eines Rechenalgorithmus, der vom Spezialisten auf der Grundlage des erworbenen Blutzuckerwertes und der zeitlichen Entwicklung des Blutzuckerspiegels in einem früheren Zeitraum bereitgestellt wird, sowie für die Anzeige der Insulindosis auf dem Display um ein Datum und eine Uhrzeit für die anschließende Messung des Blutzuckerspiegels zu definieren und den Patienten zu warnen die Messung in dem Datum und der Uhrzeit durchzuführen;
wobei das Behandlungsmanagementprogramm außerdem mindestens ein zweiten, entfernten Managementverfahren umfasst, das zur Implementierung eines zweiten, längeren Zyklus dient, wobei das entfernte Fernmanagementverfahren aus Folgen von Anweisungen zum Ablesen des Blutzuckerwertes des letzten Patienten aus dem Datenbereich besteht sowie zur periodischen Durchführung einer Prozedur, die eine Gruppe von Daten extrahiert, die für den Patienten von Bedeutung sind, welche Daten sich zumindest auf die Entwicklung der Blutzuckerwerte des Patienten und auf seine Insulindosen, die auf der Grundlage einer Berechnung durch den Rechenalgorithmus in einem vorher festgelegten Zeitraum empfohlen werden, beziehen, sowie für die Übertragung der Gruppe von bedeutungsvollen Daten an einen entfernten Computer mittels der genannten Sender- und Empfängermittel, sowie für den möglichen Empfang eines Satzes von Parametern, die auf der Grundlage einer Abweichung vom Blutzuckertrend in Bezug auf den optimalen verändert werden, sowie zum Durchführen des Rechenalgorithmus oder eines neuen Rechenalgorithmus, und um den Satz modifizierter Parameter oder den neuen Algorithmus in das Behandlungsverwaltungsprogramm einzuführen, für die Anwendung derselbe im oben genannten ersten lokalen Zyklus zugänglich ist;
wobei das lokale Managementverfahren außerdem Anweisungen zur Überwachung der seit der letzten Messung verstrichenen Zeit und für eine konsequente Wiederholung des ersten lokalen Arbeitszyklus am Datum und durch den Rechenalgorithmus berechneten Zeit, enthält, und wobei der erste, kürzere lokale Arbeitszyklus und der zweite, längere entfernte Arbeitszyklus werden asynchron und gleichzeitig ausgeführt.

2. Ein Gerät nach Anspruch 1, **dadurch gekennzeichnet, dass** das Sender- und Empfängermittel ist in die Struktur des digitalen Glucometers integriert.

3. Ein Gerät nach Anspruch 1, **dadurch gekennzeichnet, dass** in der Datenverarbeitungseinheit das Behandlungsverwaltungsprogramm mindestens eine Prozedur enthält, die darauf abzielt, den Berechnungsalgorithmus der Insulindosierung des Patienten zu implementieren basierend auf periodischen Informationen über den Blutzuckerspiegel des Patienten und die Insulindosierung, die vom Patienten in einem bestimmten Zeitraum tatsächlich eingenommen wurde.

## Revendications

1. Un dispositif pour la définition et la gestion interactive d'un traitement pour contrôler la glycémie chez un patient diabétique, ledit dispositif comprenant:
un glucomètre numérique, qu'il forme des moyens d'autosurveillance, qui mesurent la valeur glycémique dudit patient et mémorise la valeur mesurée sous la forme d'un code numérique, ledit glucomètre numérique comprenant ladite une unité de traitement, qui exécute au moins un programme de gestion du traitement;
un écran, commandé par l'unité de traitement des données et connecté électriquement à cette unité;
une pluralité de touches pour l'entrée de données ou de commandes, également reliées électriquement à l'unité de traitement des données.
ledit glucomètre numérique étant équipé de moyens de transmission et de réception, lesquels agissant par l'intermédiaire d'un réseau téléphonique mobile et sont commandé par l'unité de traitement des données pour recevoir et transmettre des données en provenance et à destination d'au moins un dispositif de réception/transmission à distance correspondant, connecté à un ordinateur qui est accessible par un spécialiste supervisant le traitement médical susmentionné du patient diabétique;
le programme de gestion du traitement comprend au moins une procédure de gestion locale mettant en oeuvre un premier cycle local plus court, ladite procédure de gestion locale comprenant des séquences d'instructions pour l'acquisition d'au moins une valeur de glycémie du patient par les moyens d'autosurveillance et pour stocker la valeur dans une zone de données de ladite unité de traitement de données, pour calculer une valeur de dose d'insuline à administrer au patient, au moyen d'un algorithme de calcul qui est fourni par le spécialiste, sur la base de la valeur de glycémie acquise et de l'évolution temporelle de la glycémie au cours d'une période précédente, pour l'affichage de la dose d'insuline sur l'écran, pour définir une date et une heure pour la mesure ultérieure de la glycémie et des instructions pour avertir le patient d'effectuer ladite mesure dans la date et à ladite heure;
ledite programme de gestion du traitement comprend en outre au moins une procédure de gestion à distance pour la mise en oeuvre d'un deuxième cycle plus long, ladite procédure de gestion à distance comprenant des séquences d'instructions pour la lecture de la dernière valeur de glycémie du patient à partir de la zone de données, pour le déroulement périodique d'une procédure extrayant un groupe de données significatives pour le patient, liées au moins à la tendance des valeurs de glycémie du patient. et à ses doses d'insuline recommandées sur la base d'un calcul effectué par l'algorithme de calcul au cours d'une période prédéterminée précédente, pour transmettre le groupe de données significatives à un ordinateur distant, qui est accessible par le spécialiste, au moyen desdits moyens de transmission et de réception, pour la réception éventuelle d'un ensemble de paramètres qui sont modifiés sur la base d'un écart par rapport à ladite tendance de la glycémie par rapport à la tendance optimale, pour exécuter l'algorithme de calcul ou un nouvel algorithme de calcul, et pour introduire ledit ensemble de paramètres modifiés ou ledit nouvel algorithme dans ledit programme de gestion de traitement, pour l'application dudit algorithme dans le premier cycle local mentionné ci-dessus;
dans lequel ladite procédure de gestion locale comprend en outre des instructions pour contrôler le temps écoulé depuis la dernière mesure et pour une réexécution conséquente du premier cycle de travail local à la date et à l'heure calculées par l'algorithme de calcul, et dans lequel le premier cycle de travail local plus court et le second cycle de travail à distance plus long sont exécutés de manière asynchrone et simultanée.

2. Dispositif selon la revendication 1, **caractérisé en ce que** lesdites moyens de transmission et de réception sont intégrés dans la structure du glucomètre numérique.

3. Dispositif selon la revendication 1, **caractérisé en ce que**, dans l'unité de traitement des données, ledit programme de gestion du traitement comprend au moins une procédure destinée à mettre en oeuvre l'algorithme de calcul de la dose d'insuline du patient, sur la base d'informations périodiques relatives au niveau de la glycémie du patient et aux doses d'insuline qui est effectivement pris par le patient au cours d'une période antérieure donnée.
